Europäisches Patentamt

European Patent Office

Office européen des brevets

(19)

(11) **EP 1 201 222 B1**

(12) **FASCICULE DE BREVET EUROPEEN**

(45) Date de publication et mention
de la délivrance du brevet:
**01.09.2004 Bulletin 2004/36**

(51) Int Cl.⁷: **A61K 7/032**

(21) Numéro de dépôt: **01402489.7**

(22) Date de dépôt: **27.09.2001**

(54) **Composition cosmétique filmogène**

Filmbildende kosmetische Zusammensetzung

Film-forming cosmetic composition

(84) Etats contractants désignés:
**AT BE CH CY DE DK ES FI FR GB GR IE IT LI LU
MC NL PT SE TR**

(30) Priorité: **27.10.2000 FR 0013876**

(43) Date de publication de la demande:
**02.05.2002 Bulletin 2002/18**

(73) Titulaire: **L'OREAL**
**75008 Paris (FR)**

(72) Inventeurs:
• **de la Poterie, Valérie**
**77820 Le Chatelet-en-Brie (FR)**
• **Collin, Nathalie**
**92330 Sceaux (FR)**
• **Piot, Bertrand**
**75009 Paris (FR)**

(74) Mandataire: **Kromer, Christophe**
**L'OREAL - D.I.P.I.**
**25-29 Quai Aulagnier**
**92600 Asnières (FR)**

(56) Documents cités:
**EP-A- 0 639 371          EP-A- 0 847 753**
**EP-A- 0 853 940          EP-A- 0 928 607**
**EP-A- 1 013 256          EP-A- 1 031 342**
**WO-A- 01/17488           US-A- 5 389 363**
**US-A- 5 866 111          US-A- 6 106 813**
**US-A- 6 113 925**

## Description

La présente invention concerne l'utilisation d'une composition cosmétique comprenant un polymère filmogène et une cire pour l'obtention d'un film présentant une bonne tenue à l'eau froide et démaquillable à l'eau chaude. L'invention a aussi pour objet un mascara ainsi qu'un procédé de maquillage ou de soin cosmétique des fibres kératiniques.

La composition pour l'utilisation selon l'invention peut être utilisée pour le maquillage ou de soin des matières kératiniques comme la peau, les cils, les sourcils, les cheveux et les ongles, notamment d'êtres humains ; elle peut se présenter sous la forme de mascara, d'eye-liner, de produit pour les lèvres, de fard à joues ou à paupières, de fond de teint, de produit de maquillage du corps, de produit anticernes, de produit pour les ongles, de composition de protection solaire, de coloration de la peau, de produit de soin de la peau. Plus spécialement, l'invention porte sur un mascara.

Par mascara, on entend une composition destinée à être appliquée sur les cils : il peut s'agir d'une composition de maquillage des cils, une base de maquillage des cils, une composition à appliquer sur un mascara, dite encore top-coat, ou bien encore une composition de traitement cosmétique des cils. Le mascara est plus particulièrement destiné aux cils d'êtres humains, mais également aux faux-cils.

Il est connu du document WO-A-95/15741 des compositions de mascaras sous forme d'émulsion cire-dans-eau comprenant des tensio-actifs. Toutefois, le film de maquillage obtenu avec ces compositions ne présente pas une bonne résistance à l'eau et le film au contact de l'eau, pendant la baignade ou la douche par exemple, se désagrège en partie en s'effritant ou bien encore en s'étalant autour de l'oeil. L'effritement du film engendre une perte sensible de l'intensité de la couleur du maquillage, obligeant ainsi la consommatrice à renouveler l'application du mascara. L'étalement du film forme, quant à lui, une auréole autour de la zone maquillée très inesthétique. Les larmes et la transpiration provoquent également ces mêmes inconvénients.

Pour favoriser la tenue à l'eau du maquillage, il est connu du document US-A-4423031 d'utiliser des polymères acryliques en dispersion aqueuse. Néanmoins, le mascara est difficile à démaquiller et nécessite l'emploi de démaquillants spécifiques à base d'huiles ou de solvants organiques. Or ces démaquillants peuvent être irritants pour l'oeil, notamment provoquer des picotements ou laisser un voile sur l'oeil, ou bien encore peuvent laisser sur la peau autour de l'oeil (paupières) un film résiduel gras inconfortable.

Pour éviter l'emploi de ces démaquillants spécifiques, il est possible d'employer de l'eau et du savon comme le décrit le document WO-A-96/33690 en proposant un mascara comprenant un polymère insoluble dans l'eau et un polymère filmogène hydrosoluble. Toutefois, l'emploi de savon peut provoquer un inconfort occulaire dû à des picotements ou au dépôt d'un voile sur l'oeil. Le savon solubilise aussi le film de maquillage qui s'étale alors autour de l'oeil et forme des auréoles inesthétiques et tache la peau.

L'emploi d'eau chaude, c'est-à-dire d'eau ayant une température supérieure ou égale à 35 °C (température mesurée à la pression atmosphérique), et notamment allant environ de 35 °C à 50 °C, permet d'éviter les inconvénients des démaquillants connus jusqu'à présents mais les compositions de mascara résistante à l'eau froide décrites précédemment ne sont pas éliminables à l'eau chaude.

Le but de la présente invention est donc de proposer une composition cosmétique démaquillable à l'eau chaude tout en présentant une bonne tenue à l'eau froide.

Les inventeurs ont découvert qu'une telle composition pouvait être obtenue en utilisant une dispersion aqueuse de particules de polymère filmogène et une microdispersion de cire.

Après application de la composition sur les matières kératiniques, notament sur les cils, le maquillage obtenu est bien résistant à l'eau froide, c'est-à-dire à une eau ayant une température inférieure ou égale à 30 °C, lors de baignade par exemple, et/ou aux larmes et/ou à la transpiration. Le maquillage s'élimine facilement avec de l'eau chaude, notamment par frottements avec un coton ou une gaze : le maquillage se décolle facilement des cils et est retiré des cils sans se fragmenter (en forme de gaine) ou sous forme de fragments ou de morceaux. Le maquillage ainsi éliminé ne s'étale pas sur la peau ce qui évite la formation d'auréoles autour de l'oeil ; la peau n'est pas tachée lors du démaquillage et reste propre. Le maquillage s'élimine très simplement avec de l'eau chaude et en particulier avec de l'eau chaude ne contenant pas d'agent détergent tel que les savons. Pour le démaquillage, l'eau chaude utilisée peut être de l'eau de robinet, de l'eau déminéralisée ou bien encore de l'eau minérale portées à une température supérieure ou égale à 35 °C, et notamment allant environ de 35 °C à 50 °C.

Il est déjà connu du document EP-A-847753 une composition susceptible d'être appliquée sur la peau, les semi-muqueuses et les muqueuses comprenant une dispersion aqueuse de particules de polymères filmogènes et une dispersion aqueuse de cires.

Le document brevet EP-A-0 853 940 décrit l'utilisation d'une composition comprenant, dans un milieu physiologiquement acceptable contenant une phase aqueuse, une microdispersion aqueuse de particules de cire et une dispersion aqueuse de particules de polymère filmogène, pour obtenir un film déposé sur les matières kératiniques résistant à l'eau froide et démaquillable à l'eau chaude savonneuse. L'eau de démaquillage contient donc un détergent, et les compositions ne sont pas réellement adaptées comme mascara.

Le document brevet WO 01/17488, opposable uniquement au titre de l'Article 54(3) CBE, décrit un mascara comprenant une dispersion aqueuse de particules de polymère filmogène, caractérisée par le fait qu'elle comprend une microdispersion aqueuse de particules de cire, ledit polymère filmogène n'étant pas un polymère colorant. Lesdites particules de polymère filmogène ont une taille inférieure ou égale à 10 microns.

De façon plus précise, l'invention a pour objet l'utilisation d'une composition comprenant, dans un milieu physiologiquement acceptable contenant une phase aqueuse, une microdispersion aqueuse de particules de cire et une dispersion aqueuse de particules de polymère filmogène, pour obtenir un film déposé sur les matières kératiniques résistant à l'eau froide et démaquillable à l'eau chaude.

L'invention a également pour objet l'utilisation d'une microdispersion aqueuse de particules de cire et d'une dispersion aqueuse de particules de polymère filmogène, dans une composition comprenant, un milieu physiologiquement acceptable contenant une phase aqueuse, pour obtenir un film déposé sur les matières kératiniques résistant à l'eau froide et démaquillable à l'eau chaude.

L'invention a aussi pour objet un mascara comprenant dans un milieu physiologiquement acceptable contenant une phase aqueuse, une microdispersion aqueuse de particules de cire et une dispersion aqueuse de particules, de polymère filmogène, lesdites particules de polymère filmogène ayant une taille supérieure ou égale à 10 nm et ledit polymère filmogène n'étant pas un polymère colorant.

L'invention a encore pour objet un procédé cosmétique de maquillage ou de soin non thérapeutique des fibres kératiniques, notamment les cils, comprenant l'application sur les fibres kératiniques d'une composition telle que définie précédemment.

L'invention a également pour objet un procédé cosmétique de démaquillage des matières kératiniques maquillées avec une composition telle que définie précédemment, comprenant au moins une étape de rinçage avec de l'eau chaude portée à une température supérieure ou égale à 35 °C des dites matières kératiniques maquillées.

Par physiologiquement acceptable, il faut comprendre un milieu compatible avec les matières kératiniques, comme un milieu cosmétique.

Le démaquillage à l'eau chaude est obtenu en utilisant une microdispersion aqueuse de cire qui rend le film plus sensible à l'eau : le film est fragilisé lors du contact avec l'eau chaude et en le frottant, par exemple avec les doigts ou bien avec un tissu ou un coton, le film se désagrège facilement ou se décolle de son support.

a) le polymère filmogène en dispersion aqueuse :

La composition selon l'invention contient un polymère filmogène se présentant sous la forme de particules en dispersion aqueuse, connue généralement sous le nom de latex ou pseudolatex.

Dans la présente demande, on entend par "polymère filmogène", un polymère apte à former à lui seul ou en présence d'un agent auxiliaire de filmification, un film continu et adhérent sur un support, notamment sur les matières kératiniques comme les cils. De préférence, le polymère filmogène en dispersion aqueuse n'est pas un polymère colorant, ce qui exclut les polymères comprenant au moins un colorant organique monomérique.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth) acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth) acrylates d'hydroxyalkyle, en particulier d'hydroxyalkyle en $C_2$-$C_6$. Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique utilisable selon l'invention, on peut citer ceux vendus sous les dénominations NEOCRYL XK-90® , NEOCRYL A-1070® , NEOCRYL A-1090®, NEOCRYL BT-62®, NEOCRYL A-1079®, NEOCRYL A-523 ® par la société AVECIA-NEORESINS, DOW LATEX 432® par la société DOW CHEMICAL, DAITOSOL 5000 AD par la société DAITO KASEY KOGYO.

Parmi les polycondensats filmogènes, on peut également citer les polyuréthanes, les polyesters, les polyesters amides, les polyesters à chaîne grasse, les polyamides, et les résines époxyesters. On utilise de préférence les polyuréthanes.

Les polyuréthanes peuvent être choisis parmi les polyuréthannes anioniques, cationiques, non-ioniques ou amphotères, les polyuréthannes-acryliques, les polyuréthannes-polyvinylpirrolidones, les polyester-polyuréthannes, les polyétherpolyuréthannes, les polyurées, les polyurée-polyuréthannes, et leurs mélanges. Le polyuréthanne filmogène peut être, par exemple, un copolymère polyuréthanne, polyurée-uréthanne, ou polyurée, aliphatique, cycloaliphatique ou aromatique, comportant seule ou en mélange :

- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Les polyuréthannes filmogènes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Comme polyuréthanne filmogène utilisable selon l'invention on peut utiliser ceux commercialisés sous les dénominations NEOREZ R-981® , NEOREZ R-974® par la société AVECIA-NEORESINS, les AVALURE UR-405® , AVALURE UR-410® , AVALURE UR-425®, AVALURE UR-450®, SANCURE 875®, SANCURE 861®, SANCURE 878®, SANCURE 2060® par la société GOODRICH, IMPRANIL 85® par la société BAYER.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.

Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO$_3$M, avec M représentant un atome d'hydrogène, un

ion ammonium $NH_4^+$ ou un ion métallique, comme par exemple un ion $Na^+$, $Li^+$, $K^+$, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion comprenant un ou plusieurs polymères filmogènes peut être préparée par l'homme du métier sur base de ses connaissances générales.

La taille des particules de polymères en dispersion aqueuse peut aller de 10 nm à 500 nm, et de préférence de 20 nm à 300 nm.

Avantageusement, on utilise un polymère filmogène ayant une reprise en eau inférieure ou égale à 50 %, de préférence inférieure ou égale à 40 %, plus préférentiellement inférieure ou égale à 30 %, et mieux inférieure ou égale à 20 %.

Selon la présente demande, on entend par "reprise en eau du polymère filmogène", le pourcentage d'eau absorbé par le polymère après 10 minutes d'immersion dans l'eau, à 20 °C. La reprise en eau est mesurée pour une couche de 300 μm d'épaisseur (avant séchage) déposée sur une plaque puis séchée pendant 24 heures à 30 °C et à 50 % d'humidité relative ; des morceaux d'environ 1 $cm^2$ découpés dans le film sec sont pesés (mesure de la masse M1) puis immergés dans l'eau pendant 10 minutes ; après immersion, le morceau de film est essuyé pour éliminer l'excédent d'eau en surface puis pesé (mesure de la masse M2). La différence M2 - M1 correspond à la quantité d'eau absorbée par le polymère.

La reprise en eau est égale à [ (M2 - M1) / M1 ] x 100 et est exprimée en pourcentage de poids d'eau par rapport au poids de polymère.

Le polymère filmogène en dispersion aqueuse peut être présent dans la composition selon l'invention en une teneur en matières sèches allant de 1 % à 60 % en poids par rapport au poids total de la composition, de préférence de 5 % à 40% en poids, et mieux de 10 % à 30 % en poids.

La composition selon l'invention peut comprendre un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

La phase aqueuse de la composition peut être constitué essentiellement d'eau. Il peut comprendre également un mélange d'eau et de solvant miscible à l'eau comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$. La phase aqueuse (eau et éventuellement le solvant organique miscible à l'eau) représente, en pratique, de 5 % à 99,4 % en poids, par rapport au poids total de la composition.

b) la microdispersion de cire :

La composition selon l'invention comprend par ailleurs une microdispersion aqueuse de particules de cire. On entend par microdispersion aqueuse de cire, une dispersion aqueuse de particules de cire, dans laquelle la taille desdites particules de cire est inférieure ou égale à environ 1 μm.

Dans la présente demande, une cire est un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

Le point de fusion de la cire peut être mesuré à l'aide d'un calorimètre à balayage différentiel (D.S.C.), par exemple le calorimètre vendu sous la dénomination DSC 30 par la société METLER. Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les microdispersions de cire sont des dispersions stables de particules colloïdales de cire, et sont notamment décrites dans "Microemulsions Theory and Practice", L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire. Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les cires susceptibles d'être utilisées dans les compositions selon l'invention sont choisies parmi les cires, solides et rigides, à température ambiante d'origine animale, végétale, minérale ou de synthèse et leurs mélanges. Les cires peuvent avoir un point de fusion allant de 30 °C à 120 °C environ, et mieux de 45 °C à 120 °C. La cire peut également présenter une dureté allant de 0,05 MPa à 15 Mpa, et de préférence allant de 3 MPa à 15 MPa. La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

Avantageusement, on utilise une microdispersion de cire polaire. On entend par cire polaire une cire contenant des composés chimiques comportant au moins un groupement polaire. Les groupements polaires sont bien connus de l'homme du métier ; il peut s'agir par exemple de groupement alcool, ester, acide carboxylique. Ne font pas partie des cires polaires les cires de polyéthylène, les cires de paraffine, les cires microcristallines, l'ozokérite, les cires de Fisher-Tropsch. En particulier, les cires polaires ont un paramètre moyen de solubilité dA de HANSEN à 25 °C tel que dA > 0 (J/cm$^3$)$^{½}$ et mieux dA > 1 (J/cm$^3$)$^{½}$.

$$\delta_u = \sqrt{\delta_p^2 + \delta_h^2}$$

où dP et dH sont respectivement les contributions polaires et de types interactions spécifiques aux paramètres de solubilité de Hansen.

La définition des solvants dans l'espace de solubilité tridimensionnel selon HANSEN est décrite dans l'article de C. M. HANSEN : "The three dimensionnal solubility parameters" J. Paint Technol. 39, 105 (1967) ;

- dH caractérise les forces d'interactions spécifiques (type liaisons hydrogène, acide/base, donneur/accepteur, etc...) ;

- dP caractérise les forces d'interactions de DEBYE entre dipôles permanents ainsi que les forces d'interactions de KEESOM entre dipôles induits et dipôles permanents.

Les paramètres dP et dH sont exprimés en (J/cm$^3$)$^{½}$.

On peut notamment citer les cires hydrocarbonées comme la cire d'abeilles, la cire de lanoline, et les cires d'insectes de Chine; la cire de riz, la cire de Carnauba, la cire de Candellila, la cire d'Ouricurry, la cire de fibres de liège, la cire de canne à sucre, la cire du Japon et la cire de sumac; la cire de montan, et les copolymères cireux ainsi que leurs esters.

On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.

On peut encore citer les cires de silicone, les cires fluorées.

On utilise de préférence la cire de carnauba, la cire d'abeille, la cire de Candelilla.

Il est également possible d'utiliser des mélanges commerciaux de cires auto-émulsionnables contenant une cire et des tensioactifs. Ces mélanges commerciaux permettent de préparer des microdispersions de cires par simple addition d'eau.

La composition selon l'invention peut comprendre, de préférence, de 0,1 % à 50 % en poids de matière sèche de cire sous forme de microdispersion de cire (appelée première cire), notamment de 1 % à 30 % en poids, et mieux de 5 % à 20 % en poids.

La composition peut également comprendre une quantité suffisante de tensioactif pour permettre d'obtenir une microdispersion de cire, ainsi qu'une composition finale, stable. Notamment, elle peut comprendre 0,01 à 5% en poids de tensioactif usuel, pouvant être choisi parmi les composés suivants :

- les tensioactifs anioniques, notamment des sels d'acides gras éventuellement insaturés, ayant par exemple 12 à 18 atomes de carbone; des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient 6-18 atomes de carbone; les éthers-sulfates.
- les tensioactifs non ioniques, notamment des tensioactifs polyalcoxylés et/ou polyglycérolés, et en particulier des acides gras ou amides d'acide gras; des alcools gras ou des alkylphénols; les esters d'acides gras et de polyols; les alcanediols et les alkyléthers d'alcanediols. On peut citer également les alkylcarbamates de triglycérol, les dérivés oxyéthylénés ou propoxylés des alcools de lanoline, des acides gras de la lanoline, ou de leur mélanges.
- les tensioactifs cationiques, notamment les dérivés d'ammonium quaternaire.

La cire ou mélange de cires, peut être associé à un ou plusieurs additifs gras (huileux et/ou pâteux). On peut notamment citer les huiles végétales comme l'huile de tournesol, l'huile de jojoba; les huiles minérales comme l'huile de paraffine; les huiles de silicones; la vaseline, la lanoline; les huiles fluorées; les huiles hydrocarbonées à groupement perfluoré; les esters d'alcools gras.

Il est possible d'introduire en outre dans la phase cireuse microparticulaire des ingrédients actifs liposolubles, tels que des filtres U.V., des vitamines liposolubles, des actifs cosmétiques liposolubles.

Avantageusement, la composition peut comprendre la microdispersion de cire et le polymère filmogène en dispersion aqueuse en un rapport pondéral polymère filmogène / cire microdispersée allant de 50/50 à 95/5, et mieux allant de 60/40 à 80/20.

c) les additifs :

La composition peut comprendre en outre, en plus de la microdispersion de cire, une cire additionnelle sous forme de particules de taille supérieure ou égale à 1 μm, de préférence supérieure ou égale à 1,3 μm, dispersées dans la phase aqueuse. Cette cire additionnelle ne se présente donc pas sous la forme d'une microdispersion aqueuse de particules de cire telle que définie précédemment. En particulier, la taille moyenne des particules de la cire additionnelle peut aller de 1 μm à 10 μm, et de préférence de 1,3 μm à 5 μm.

La cire additionnelle permet d'obtenir un maquillage épais des cils ; on dit alors que le mascara est chargeant. La composition comprenant la cire additionnelle peut donc être utilisée pour épaissir les fibres kératiniques, notamment les cils. La cire additionnelle peut être choisie parmi les cires citées précédemment et peut être présente en une teneur allant de 0 % à 30 % en poids (notamment 0,1 % à 30 %), par rapport au poids total de la composition, de préférence allant de 1 % à 25 %, et mieux de 5 % à 20 %.

Avantageusement, le polymère filmogène et la cire additionnelle peuvent être présents dans la composition selon un rapport pondéral polymère filmogène / cire additionelle allant de 40/60 à 95/5, de préférence de 55/45 à 80/20.

La phase aqueuse de la composition peut comprendre, en outre, un polymère filmogène additionnel hydrosoluble, notamment présent en une teneur allant de 0,01 % à 5 % en poids, par rapport au poids total de la composition.

Comme polymère hydrosoluble, on peut notamment citer :

- les polymères cellulosiques hydrosolubles comme l'hydroxyéthyl cellulose, l'hydroxypropyl cellulose, la carboxyméthyl cellulose, l'hydroxypropyl éthyl cellulose, l'éthyl hydroxyéthyl cellulose
- les dérivés de kératine, tels que les hydrolysats de kératine et les kératines sulfoniques ;
- les dérivés de chitine ou de chitosane anioniques, cationiques, amphotères ou non-ioniques, et notamment l'hydroxy propyl chitosane,
- les dérivés de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylate;
- les alcools polyvinyliques et les polyvinylpyrrolidones,
- les copolymères vinyliques, tels que les copolymères de l'éther méthylvinylique et de l'anhydride malique, ou le copolymère de l'acétate de vinyle et de l'acide crotonique ;
- les polyéthylèneglycols,

- les polymères d'origine naturelle, éventuellement modifiés, tels que :

  . les gommes arabiques, la gomme de guar, les dérivés du xanthane, la gomme de karaya ;
  . les alginates et les carraghénates ;
  . les glycoaminoglycanes, l'acide hyaluronique et ses dérivés ;
  . la résine shellac, la gomme de sandaraque, les dammars, les élémis, les copals ;
  . l'acide désoxyribonucléïque.

La composition peut également comprendre au moins une matière colorante comme les composés pulvérulents, par exemple à raison de 0,01 à 50 % du poids total de la composition. Les composés pulvérulents peuvent être choisis parmi les pigments et/ou les nacres habituellement utilisés dans les compositions cosmétiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition peut également comprendre des charges qui peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de Nylon (Orgasol de chez Atochem), de poly-β-alanine et de polyéthylène, le Téflon, la lauroyllysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également contenir des ingrédients couramment utilisés en cosmétique, tels que les oligo-éléments, les adoucissants, les séquestrants, les parfums, les huiles, les silicones, les épaississants, les vitamines, les protéines, les céramides, les plastifiants, les agents de coalescence, les agents de cohésion ainsi que les agents alcalinisants ou acidifiants habituellement utilisés dans le domaine cosmétique, les émollients, les conservateurs.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée selon les méthodes usuelles des domaines considérés.

L'invention est illustrée plus en détail dans les exemples suivants.

## Exemple 1 :

On a préparé une microdispersion de cire de carnauba ayant la composition-suivante :

- Cire de carnauba          27 g
- Monostéarate de glycéryle polyoxyéthyléné (30 OE)
  ( TAGAT S de GOLDSCHMIDT)          6,75 g
- Ethanol          10 g
- Eau          qsp          100 g

On a chauffé à 90 °C la cire et le tensioactif en homogénéisant le mélange sous agitation modérée. Puis on a incorporé l'eau chauffée à 90 °C en continuant d'agiter. On a refroidi à température ambiante et ajouté l'éthanol pour obtenir une microdispersion de cire ayant un diamètre moyen de particules d'environ 170 nm.

## Exemple 2 :

On a préparé un mascara ayant la composition suivante :

- Polyuréthane en dispersion aqueuse vendu sous la dénomination AVALURE UR 425 par la société GOODRICH à 49 % en poids de matières actives          14 g MA
- Microdispersion de cire de l'exemple 1          31,5 g
- Cire d'abeille          10 g
- Agent épaississant          1,9 g
- Ethanol          7 g
- Propylène glycol          5 g
- Pigments          5 g

- Conservateurs     qs
- Eau     qsp     100 g

Le mascara s'applique facilement sur les cils et forme un maquillage résistant à l'eau froide. Il se démaquille facilement avec de l'eau chaude (40 °C).

**Exemple 3 :**

On a préparé un mascara ayant la composition suivante :

- Sulfopolyester vendu sous la dénomination EASTMAN AQ 55 S par la société EASTMAN     20,8 g MA
- Microdispersion de cire de l'exemple 1     34,5 g
- Hydroxyéthylcellulose     0,9 g
- Propylène glycol     5 g
- Pigments     7 g
- Conservateurs     qs
- Eau     qsp     100 g

**Revendications**

1. Utilisation d'une composition comprenant, dans un milieu physiologiquement acceptable contenant une phase aqueuse, une microdispersion aqueuse de particules de cire et une dispersion aqueuse de particules de polymère filmogène, pour obtenir un film déposé sur les matières kératiniques résistant à l'eau froide et démaquillable avec de l'eau chaude ne contenant pas d'agent détergent.

2. Utilisation d'une microdispersion aqueuse de particules de cire et d'une dispersion aqueuse de particules de polymère filmogène, dans une composition comprenant, un milieu physiologiquement acceptable contenant une phase aqueuse, pour obtenir un film déposé sur les matières kératiniques résistant à l'eau froide et démaquillable avec de l'eau chaude ne contenant pas d'agent détergent.

3. Utilisation d'une composition comprenant, dans un milieu physiologiquement acceptable contenant une phase aqueuse, une microdispersion aqueuse de particules de cire et une dispersion aqueuse de particules de polymère filmogène, pour obtenir un film déposé sur les fibres kératiniques résistant à l'eau froide et démaquillable avec de l'eau chaude.

4. Utilisation selon l'une des revendication 1 à 3, **caractérisée par le fait que** les particules de polymère filmogène ont une taille allant de 10 à 500 nm, et de préférence allant de 20 à 300 nm.

5. Utilisation selon l'une quelconque des revendications précédentes, **caractérisée par le fait que** le polymère filmogène en dispersion aqueuse est choisi parmi les polymères radicalaires, les polycondensats, les polymères d'origine naturelle.

6. Utilisation selon l'une quelconque des revendications précédentes, **caractérisée par le fait que** le polymère filmogène en dispersion aqueuse est choisi dans le groupe formé par les polymères vinyliques, les polyuréthanes, les polyesters, les polyamides.

7. Utilisation selon l'une quelconque des revendications précédentes, **caractérisée par le fait que** le polymère filmogène en dispersion aqueuse est un polyuréthane.

8. Utilisation selon l'une quelconque des revendications précédentes, **caractérisée par le fait que** le polymère filmogène a une reprise en eau inférieure ou égale à 50 % , de préférence inférieure ou égale à 40 %, et mieux inférieure ou égale à 30 %.

9. Utilisation selon l'une quelconque des revendications précédentes, **caractérisée par le fait que** le polymère filmogène en dispersion aqueuse est présent en une teneur allant de 1 % à 60 % en poids, par rapport au poids total de la composition, de préférence allant de 5 % à 40 % en poids, et mieux de 10 % à 30 % en poids.

10. Utilisation selon l'une quelconque des revendications précédentes, **caractérisée par le fait que** la microdispersion de cire comprend des particules de cires ayant une taille moyenne inférieure à 1 μm, de préférence inférieure à 0,5 μm.

11. Utilisation selon l'une quelconque des revendications précédentes, **caractérisée par le fait que** la cire a un point de fusion allant de 30 °C à 120 °C, et de préférence allant de 45 °C à 120 °C.

12. Utilisation selon l'une quelconque des revendications précédentes, **caractérisée par le fait que** la cire de la microdispersion est une cire polaire.

13. Utilisation selon l'une quelconque des revendications précédentes, **caractérisée par le fait que** la cire est choisie dans le groupe formé par la cire d'abeilles, la cire de lanoline, les cires d'insectes de Chine; la cire de riz, la cire de Carnauba, la cire de Candellila, la cire d'Ouricurry, la cire de fibres de liège, la cire de canne à sucre, la cire du Japon et la cire de sumac; la cire de montan, les cires obtenues par hydrogénation catalytique d'huiles animales ou végétales ayant des chaînes grasses, linéaires ou ramifiées, en C8-C32; l'huile de jojoba hydrogénée, l'huile de tournesol hydrogénée, l'huile de ricin hydrogénée, l'huile de coprah hydrogénée, l'huile de lanoline hydrogénée; les cires de silicone; leurs mélanges.

**14.** Utilisation selon l'une quelconque des revendications précédentes, **caractérisée par le fait que** la cire a une dureté allant de 0,05 MPa à 15 MPa, et de préférence allant de 3 MPa à 15 MPa.

**15.** Utilisation selon l'une quelconque des revendications précédentes, **caractérisée par le fait que** la cire de ladite microdispersion est présente en une teneur en matière sèche allant de 0,1 % à 50 % en poids, par rapport au poids total de la composition, de préférence allant de 1 % à 30% en poids, et mieux allant de 5 % à 20 % en poids.

**16.** Utilisation selon l'une quelconque des revendications précédentes, **caractérisée par** le fait les particules de la microdispersion de cire comprennent en outre des additifs gras huileux et/ou pâteux et/ou un additif/actif liposoluble.

**17.** Utilisation selon l'une des revendications précédentes, **caractérisée par le fait que** la composition comprend en outre au moins un tensioactif.

**18.** Utilisation selon l'une quelconque des revendications précédentes, **caractérisée par le fait que** le polymère filmogène en dispersion aqueuse et la microdispersion de cire sont présents dans la composition selon un rapport pondéral polymère filmogène / cire microdispersée allant de 50/50 à 95/5, de préférence de 60/40 à 80/20.

**19.** Utilisation selon l'une quelconque des revendications précédentes, **caractérisée par le fait que** la composition comprend en outre, un polymère filmogène additionnel hydrosoluble.

**20.** Utilisation selon l'une quelconque des revendications précédentes, **caractérisée par le fait que** la composition comprend une cire additionelle sous forme de particules de taille supérieure ou égale à 1 μm dispersée dans la phase aqueuse.

**21.** Utilisation selon l'une quelconque des revendications précédentes, **caractérisée par le fait que** la composition comprend en outre un additif choisi dans le groupe formé par les pigments, les nacres, les charges, les oligo-éléments, les adoucissants, les séquestrants, les parfums, les huiles, les silicones, les épaississants, les vitamines, les protéines, les céramides, les plastifiants, les agents de coalescence, les agents de cohésion, les agents alcalinisants, les agents acidifiants, les émollients, les conservateurs.

**22.** Mascara comprenant une dispersion aqueuse de particules de polymère filmogène, lesdites particules de polymère filmogène ayant une taille allant de 10 à 500 nm, **caractérisée par le fait qu'**elle comprend une microdispersion aqueuse de particules de cire, et ledit polymère filmogène n'étant pas un polymère colorant.

**23.** Mascara selon la revendication 22, **caractérisé par le fait que** les particules de polymère filmogène ont une taille allant de 20 à 300 nm.

**24.** Mascara selon la revendication 22 ou 23, **caractérisé par le fait que** le polymère filmogène en dispersion aqueuse est choisi parmi les polymères radicalaires, les polycondensats, les polymères d'origine naturelle.

**25.** Mascara selon l'une quelconque des revendications 22 à 24, **caractérisé par le fait que** le polymère filmogène est choisi parmi les polymères vinyliques, les polyuréthanes, les polyesters, les polyamides.

**26.** Mascara selon l'une quelconque des revendications 22 à 25, **caractérisé par le fait que** le polymère filmogène en dispersion aqueuse est un polyuréthane.

**27.** Mascara selon l'une quelconque des revendications 22 à 26, **caractérisé par le fait que** le polymère filmogène a une reprise en eau inférieure ou égale à 50 % , de préférence inférieure ou égale à 40 %, et mieux inférieure ou égale à 30 %.

**28.** Mascara selon l'une quelconque des revendications 22 à 27, **caractérisé par le fait que** le polymère filmogène en dispersion aqueuse est présent en une teneur allant de 1 % à 60 % en poids, par rapport au poids total de la composition, de préférence de 5 % à 40 % en poids, et mieux de 10 % à 30 % en poids.

**29.** Mascara selon selon l'une quelconque des revendications 22 à 28, **caractérisé par le fait que** la microdispersion de cire comprend des particules de cires ayant une taille moyenne inférieure à 1 μm, de préférence inférieure à 0,5 μm.

**30.** Mascara selon l'une quelconque des revendications 22 à 29, **caractérisé par le fait que** la cire a un point de fusion allant de 30 °C à 120 °C, et de préférence allant de 45 °C à 120°C.

**31.** Mascara selon l'une quelconque des revendications 22 à 30, **caractérisé par le fait que** la cire de la microdispersion est une cire polaire.

**32.** Mascara selon l'une quelconque des revendications 22 à 31, **caractérisé par le fait que** la cire est choisie dans le groupe formé par la cire d'abeilles, la cire de lanoline, les cires d'insectes de Chine; la cire de riz, la cire de Carnauba, la cire de Candellila, la cire d'Ouricurry, la cire de fibres de liège, la cire de canne à sucre, la cire du Japon et la cire de sumac; la cire de montan, les cires obtenues par hydrogénation catalytique d'huiles animales ou végétales ayant des chaînes grasses, linéaires ou ramifiées, en C8-C32; l'huile de jojoba hydrogénée, l'huile de tournesol hydrogénée, l'huile de ricin hydrogénée, l'huile de coprah hydrogénée, l'huile de lanoline hydrogénée; les cires de silicone; leurs mélanges.

33. Mascara selon l'une quelconque des revendications 22 à 32, **caractérisé par le fait que** la cire a une dureté allant de 0,05 MPa à 15 MPa, et de préférence allant de 6 MPa à 15 MPa.

34. Mascara selon l'une quelconque des revendications 22 à 33, **caractérisé par le fait que** la cire de ladite microdispersion est présente en une teneur en matière sèche allant de 0,1 % à 50 % en poids, par rapport au poids total de la composition, de préférence allant de 1 % à 30% en poids, et mieux allant de 5 % à 20 % en poids.

35. Mascara selon l'une quelconque des revendications 22 à 34, dans laquelle les particules de la microdispersion de cire comprennent en outre des additifs gras huileux et/ou pâteux et/ou un additif/actif liposoluble.

36. Mascara selon l'une des revendications 22 à 35, comprenant en outre au moins un tensioactif.

37. Mascara selon l'une quelconque des revendications 22 à 36, **caractérisé par le fait que** le polymère filmogène en dispersion aqueuse et la microdispersion de cire sont présents dans la composition selon un rapport pondéral polymère filmogène / cire microdispersée allant de 50/50 à 95/5, de préférence de 60/40 à 80/20.

38. Mascara selon l'une quelconque des revendications 22 à 37 **caractérisé par le fait que** le mascara comprend en outre, un polymère filmogène additionnel hydrosoluble.

39. Mascara selon l'une quelconque des revendications 22 à 38, **caractérisé par le fait qu'**elle comprend en outre un additif choisi dans le groupe formé par les pigments, les nacres, les charges, les oligo-éléments, les adoucissants, les séquestrants, les parfums, les huiles, les silicones, les épaississants, les vitamines, les protéines, les céramides, les plastifiants, les agents de coalescence, les agents de cohésion, les agents alcalinisants, les agents acidifiants, les émollients, les conservateurs.

40. Procédé de maquillage ou de soin non thérapeutique des fibres kératiniques, notamment des cils, **caractérisé par le fait qu'**il comprend l'application sur les fibres kératiniques d'un mascara selon l'une quelconque des revendications 22 à 39.

**Patentansprüche**

1. Verwendung einer Zusammensetzung, die in einem physiologisch akzeptablen Medium, das eine wässerige Phase enthält, eine wässerige Mikrodispersion von Wachspartikeln und eine wässerige Dispersion von Partikeln eines filmbildenden Polymers enthält, um einen auf keratinischen Materialien abgeschiedenen Film zu erhalten, der gegenüber kaltem Wasser beständig ist und mit warmem Wasser, das keine Detergentien enthält, entfernt werden kann.

2. Verwendung einer wässerigen Mikrodispersion von Wachspartikeln und einer wässerigen Dispersion von Partikeln eines filmbildenden Polymers in einer Zusammensetzung, die ein physiologisch akzeptables, eine wässerige Phase enthaltendes Medium enthält, um einen auf keratinischen Materialien abgeschiedenen Film zu erhalten, der gegenüber kaltem Wasser beständig ist und mit warmem Wasser, das keine Detergentien enthält, abgenommen werden kann.

3. Verwendung einer Zusammensetzung, die in einem physiologisch akzeptablen Medium, das eine wässerige Phase enthält, eine wässerige Mikrodispersion von Wachspartikeln und eine wässerige Dispersion von Partikeln eines filmbildenden Polymers enthält, um einen auf Keratinfasern abgeschiedenen Film zu erhalten, der gegenüber kaltem Wasser beständig ist und mit warmem Wasser entfernt werden kann.

4. Verwendung nach einem der Ansprüche 1 bis 3, **dadurch gekennzeichnet, dass** die Partikel des filmbildenden Polymers eine Größe von 10 bis 500 nm und vorzugsweise von 20 bis 300 nm aufweisen.

5. Verwendung nach einem der vorhergehenden Ansprüche, **dadurch gekennzeichnet, dass** das in wässeriger Dispersion vorliegende filmbildende Polymer unter den durch radikalische Polymerisation hergestellten Polymeren, den Polykondensaten und den Polymeren natürlichen Ursprungs ausgewählt ist.

**6.** Verwendung nach einem der vorhergehenden Ansprüche, **dadurch gekennzeichnet, dass** das in wässeriger Dispersion vorliegende filmbildende Polymer unter den Vinylpolymeren, den Polyurethanen, den Polyestern und den Polyamiden ausgewählt ist.

**7.** Verwendung nach einem der vorhergehenden Ansprüche, **dadurch gekennzeichnet, dass** es sich bei dem in wässeriger Dispersion vorliegenden filmbildenden Polymer um ein Polyurethan handelt.

**8.** Verwendung nach einem der vorhergehenden Ansprüche, **dadurch gekennzeichnet, dass** der Wert der Wasseraufnahme des filmbildenden Polymers kleiner oder gleich 50 %, vorzugsweise kleiner oder gleich 40 % und besser noch kleiner oder gleich 30 % ist.

**9.** Verwendung nach einem der vorhergehenden Ansprüche, **dadurch gekennzeichnet, dass** das in wässeriger Dispersion befindliche filmbildende Polymer in einem Mengenanteil von 1 bis 60 Gew.-%, vorzugsweise von 5 bis 40 Gew.-% und besser noch von 10 bis 30 Gew.-%, bezogen auf das Gesamtgewicht der Zusammensetzung, vorliegt.

**10.** Verwendung nach einem der vorhergehenden Ansprüche, **dadurch gekennzeichnet, dass** die Wachs-Mikrodispersion Wachspartikel mit einer mittleren Größe von kleiner 1 μm und vorzugsweise kleiner 0,5 μm enthält.

**11.** Verwendung nach einem der vorhergehenden Ansprüche, **dadurch gekennzeichnet, dass** das Wachs einen Schmelzpunkt im Bereich von 30 bis 120 °C und vorzugsweise im Bereich von 45 bis 120 °C aufweist.

**12.** Verwendung nach einem der vorhergehenden Ansprüche, **dadurch gekennzeichnet, dass** es sich bei dem Wachs der Mikrodispersion um ein polares Wachs handelt.

**13.** Verwendung nach einem der vorhergehenden Ansprüche, **dadurch gekennzeichnet, dass** das Wachs unter Bienenwachs, Lanolinwachs, Chinesischem Wachs, Reiswachs, Carnaubawachs, Candellilawachs, Ouricurywachs, Korkfaserwachs, Zuckerrohrwachs, Japanwachs und Sumachwachs, Montanwachs, Wachsen, die durch katalytische Hydrierung von tierischen oder pflanzlichen Ölen mit geradkettigen oder verzweigten $C_{8-32}$-Fettketten erhalten werden, hydriertem Jojobaöl, hydriertem Sonnenblumenöl, hydriertem Ricinusöl, hydriertem Kopraöl und hydriertem Lanolinöl, Siliconwachsen und den Gemischen dieser Verbindungen ausgewählt ist.

**14.** Verwendung nach einem der vorhergehenden Ansprüche, **dadurch gekennzeichnet, dass** das Wachs eine Härte von 0,05 bis 15 MPa und vorzugsweise von 3 bis 15 MPa aufweist.

**15.** Verwendung nach einem der vorhergehenden Ansprüche, **dadurch gekennzeichnet, dass** das Wachs der Mikrodispersion in einem Trockensubstanzgehalt von 0,1 bis 50 Gew.-%, vorzugsweise von 1 bis 30 Gew.-% und besser noch von 5 bis 20 Gew.-%, bezogen auf das Gesamtgewicht der Zusammensetzung, vorliegt.

**16.** Verwendung nach einem der vorhergehenden Ansprüche, **dadurch gekennzeichnet, dass** die Partikel der Wachs-Mikrodispersion ferner ölige und/oder pastöse Fettsubstanzen als Zusatz und/oder einen fettlöslichen Zusatzstoff/Wirkstoff enthalten.

**17.** Verwendung nach einem der vorhergehenden Ansprüche, **dadurch gekennzeichnet, dass** die Zusammensetzung ferner mindestens einen grenzflächenaktiven Stoff enthält.

**18.** Verwendung nach einem der vorhergehenden Ansprüche, **dadurch gekennzeichnet, dass** das in wässeriger Dispersion vorliegende filmbildende Polymer und die Mikrodispersion von Wachs in der Zusammensetzung in einem Gewichtsverhältnis filmbildendes Polymer/mikrodispergiertes Wachs von 50/50 bis 95/5 und vorzugsweise von 60/40 bis 80/20 vorliegen.

**19.** Verwendung nach einem der vorhergehenden Ansprüche, **dadurch gekennzeichnet, dass** die Zusammensetzung ferner ein wasserlösliches zusätzliches filmbildendes Polymer enthält.

**20.** Verwendung nach einem der vorhergehenden Ansprüche, **dadurch gekennzeichnet, dass** die Zusammensetzung ein zusätzliches Wachs in Form von Partikeln mit einer Größe von mindestens 1 μm enthält, das in der wässerigen Phase dispergiert vorliegt.

**21.** Verwendung nach einem der vorhergehenden Ansprüche, **dadurch gekennzeichnet, dass** die Zusammensetzung ferner einen Zusatzstoff enthält, der unter den Pigmenten, Perlglanzpigmenten, Füllstoffen, Spurenelementen, reizlindernden Mitteln, Maskierungsmitteln, Parfums, Ölen, Siliconen, Verdickungsmitteln, Vitaminen, Proteinen, Ceramiden, Weichmachern, Koaleszenzmitteln, Kohäsionsmitteln, Mitteln zum Alkalischmachen, Mitteln zum Ansäuern, Emollientien und Konservierungsstoffen ausgewählt ist.

**22.** Mascara, die eine wässerige Dispersion von Partikeln eines filmbildenden Polymers enthält, wobei die Partikel des filmbildenden Polymers eine Größe von 10 bis 500 nm aufweisen, **dadurch gekennzeichnet, dass** sie eine wässerige Mikrodispersion von Wachspartikeln enthält, wobei das filmbildende Polymer kein färbendes Polymer ist.

23. Mascara nach Anspruch 22, **dadurch gekennzeichnet, dass** die Partikel des filmbildenden Polymers eine Größe von 20 bis 300 nm aufweisen.

24. Mascara nach Anspruch 22 oder 23, **dadurch gekennzeichnet, dass** das in wässeriger Dispersion vorliegende filmbildende Polymer unter den durch radikalische Polymerisation hergestellten Polymeren, den Polykondensaten und den Polymeren natürlichen Ursprungs ausgewählt ist.

25. Mascara nach einem der Ansprüche 22 bis 24, **dadurch gekennzeichnet, dass** das filmbildende Polymer unter den Vinylpolymeren, den Polyurethanen, den Polyestern und den Polyamiden ausgewählt ist.

26. Mascara nach einem der Ansprüche 22 bis 25, **dadurch gekennzeichnet, dass** das in wässeriger Dispersion vorliegende filmbildende Polymer ein Polyurethan ist.

27. Mascara nach einem der Ansprüche 22 bis 26, **dadurch gekennzeichnet, dass** der Wert der Wasseraufnahme des filmbildenden Polymers kleiner oder gleich 50 %, vorzugsweise kleiner oder gleich 40 % und besser noch kleiner oder gleich 30 % ist.

28. Mascara nach einem der Ansprüche 22 bis 27, **dadurch gekennzeichnet, dass** das in wässeriger Dispersion vorliegende filmbildende Polymer in einem Mengenanteil von 1 bis 60 Gew.-%, vorzugsweise von 5 bis 40 Gew.-% und besser noch von 10 bis 30 Gew.-%, bezogen auf das Gesamtgewicht der Zusammensetzung, vorliegt.

29. Mascara nach einem der Ansprüche 22 bis 28, **dadurch gekennzeichnet, dass** die Wachs-Mikrodispersion Wachspartikel mit einer mittleren Größe von kleiner 1 μm und vorzugsweise kleiner 0,5 μm enthält.

30. Mascara nach einem der Ansprüche 22 bis 29, **dadurch gekennzeichnet, dass** das Wachs einen Schmelzpunkt im Bereich von 30 bis 120 °C und vorzugsweise von 45 bis 120 °C aufweist.

31. Mascara nach einem der Ansprüche 22 bis 30, **dadurch gekennzeichnet, dass** das Wachs der Mikrodispersion ein polares Wachs ist.

32. Mascara nach einem der Ansprüche 22 bis 31, **dadurch gekennzeichnet, dass** das Wachs unter Bienenwachs, Lanolinwachs, Chinesischem Wachs, Reiswachs, Carnaubawachs, Candellilawachs, Ouricurywachs, Korkfaserwachs, Zuckerrohrwachs, Japanwachs und Sumachwachs, Montanwachs, Wachsen, die durch katalytische Hydrierung von tierischen oder pflanzlichen Ölen mit geradkettigen oder verzweigten $C_{8-32}$-Fettketten erhalten werden, hydriertem Jojobaöl, hydriertem Sonnenblumenöl, hydriertem Ricinusöl, hydriertem Kopraöl und hydriertem Lanolinöl, Siliconwachsen und den Gemischen dieser Verbindungen ausgewählt ist.

33. Mascara nach einem der Ansprüche 22 bis 32, **dadurch gekennzeichnet, dass** das Wachs eine Härte von 0,05 bis 15 MPa und vorzugsweise von 6 bis 15 MPa aufweist.

34. Mascara nach einem der Ansprüche 22 bis 33, **dadurch gekennzeichnet, dass** das Wachs der Mikrodispersion in einem Trockensubstanzgehalt von 0,1 bis 50 Gew.-%, vorzugsweise von 1 bis 30 Gew.-% und besser noch von 5 bis 20 Gew.-%, bezogen auf das Gesamtgewicht der Zusammensetzung, vorliegt.

35. Mascara nach einem der Ansprüche 22 bis 34, worin die Partikel der Wachs-Mikrodispersion außerdem ölige und/oder pastöse Fettsubstanzen als Zusatz und/oder einen fettlöslichen Zusatzstoff/ Wirkstoff enthalten.

36. Mascara nach einem der Ansprüche 22 bis 35, die ferner mindestens einen grenzflächenaktiven Stoff enthält.

37. Mascara nach einem der Ansprüche 22 bis 36, **dadurch gekennzeichnet, dass** das in wässeriger Dispersion befindliche filmbildende Polymer und die Wachs-Mikrodispersion in der Zusammensetzung in einem Gewichtsverhältnis filmbildendes Polymer/ mikrodispergiertes Wachs von 50/50 bis 95/5 und vorzugsweise von 60/40 bis 80/20 vorliegt.

38. Mascara nach einem der Ansprüche 22 bis 37, **dadurch gekennzeichnet, dass** die Mascara ferner ein wasserlösliches zusätzliches filmbildendes Polymer enthält.

39. Mascara nach einem der Ansprüche 22 bis 38, **dadurch gekennzeichnet, dass** sie ferner einen Zusatzstoff enthält, der unter den Pigmenten, Perlglanzpigmenten, Füllstoffen, Spurenelementen, reizlindemden Mitteln, Maskierungsmitteln, Parfums, Ölen, Siliconen, Verdickungsmitteln, Vitaminen, Proteinen, Ceramiden, Weichmachern, Koaleszenzmitteln, Kohäsionsmitteln, Mitteln zum Alkalischmachen, Mitteln zum Ansäuern, Emollientien und Konservierungsstoffen ausgewählt ist.

40. Nichttherapeutisches Verfahren zum Schminken oder zur Pflege von Keratinfasern und insbesondere der Wimpern, **dadurch gekennzeichnet, dass** es die Applikation einer Mascara nach einem der Ansprüche 22 bis 39 auf die Keratinfasern beinhaltet.


**Claims**

1. Use of a composition comprising, in a physiologically acceptable medium containing an aqueous phase, an aqueous microdispersion of particles of wax and an aqueous dispersion of particles of film-forming polymer, in order to obtain a film deposited on the keratinous materials which is resistant to cold water and which, when used as make-up, can be removed with hot water not containing any detergent.

2. Use of an aqueous microdispersion of particles of wax and of an aqueous dispersion of particles of film-forming polymer, in a composition comprising a physiologically acceptable medium containing an aqueous phase, in order to obtain a film deposited on the keratinous materials which is resistant to cold water and which, when used as make-up, can be removed with hot water not containing any detergent.

3. Use of a composition comprising, in a physiologically acceptable medium containing an aqueous phase, an aqueous microdispersion of particles of wax and an aqueous dispersion of particles of film-forming polymer, in order to obtain a film deposited on the keratinous fibres which is resistant to cold water and which, when used as make-up, can be removed with hot water.

4. Use according to one of Claims 1 to 3, **characterized in that** the particles of film-forming polymer have a size ranging from 10 to 500 nm, and preferably ranging from 20 to 300 nm.

5. Use according to any one of the preceding claims, **characterized in that** the film-forming polymer in aqueous dispersion is chosen from free-radical polymers, polycondensates and polymers of natural origin.

6. Use according to any one of the preceding claims, **characterized in that** the film-forming polymer in aqueous dispersion is chosen from the group formed by vinyl polymers, polyurethanes, polyesters and polyamides.

7. Use according to any one of the preceding claims, **characterized in that** the film-forming polymer in aqueous dispersion is a polyurethane.

8. Use according to any one of the preceding claims, **characterized in that** the film-forming polymer has a water uptake of less than or equal to 50%, preferably of less than or equal to 40% and better still of less than or equal to 30%.

9. Use according to any one of the preceding claims, **characterized in that** the film-forming polymer in aqueous dispersion is present in an amount ranging from 1% to 60% by weight relative to the total weight of the composition, preferably ranging from 5% to 40% by weight, and better still from 10% to 30% by weight.

10. Use according to any one of the preceding claims, **characterized in that** the microdispersion of wax comprises particles of waxes having a mean size of less than 1 μm, preferably of less than 0.5 μm.

11. Use according to any one of the preceding claims, **characterized in that** the wax has a melting point ranging from 30°C to 120°C, and preferably ranging from 45°C to 120°C.

12. Use according to any one of the preceding claims, **characterized in that** the wax of the microdispersion is a polar wax.

13. Use according to any one of the preceding claims, **characterized in that** the wax is chosen from the group formed by beeswax, lanolin wax, Chinese waxes; rice wax, Carnauba wax, candelilla wax, ouricury wax, cork fibre wax, sugarcane wax, Japan wax and sumac wax; montan wax, the waxes obtained by catalytic hydrogenation of animal or vegetable oils having linear or branched $C_8$-$C_{32}$ fatty chains; hydrogenated jojoba oil, hydrogenated sunflower oil, hydrogenated castor oil, hydrogenated copra oil and hydrogenated lanolin oil; silicone waxes; mixtures thereof.

14. Use according to any one of the preceding claims, **characterized in that** the wax has a hardness ranging from 0.05 MPa to 15 MPa, and preferably ranging from 3 MPa to 15 MPa.

15. Use according to any one of the preceding claims, **characterized in that** the wax of the said microdispersion is present in a dry matter content ranging from 0.1% to 50% by weight, relative to the total weight of the composition, preferably ranging from 1% to 30% by weight, and better still ranging from 5% to 20% by weight.

16. Use according to any one of the preceding claims, **characterized in that** the particles of the microdispersion of wax comprise, in addition, oily and/or pasty fatty additives and/or a fat-soluble additive/active agent.

17. Use according to one of the preceding claims, **characterized in that** the composition comprises, in addition, at least one surfactant.

18. Use according to any one of the preceding claims, **characterized in that** the film-forming polymer in aqueous dispersion and the microdispersion of wax are present in the composition in a film-forming polymer/microdispersed wax weight ratio ranging from 50/50 to 95/5, preferably 60/40 to 80/20.

19. Use according to any one of the preceding claims, **characterized in that** the composition comprises, in addition, an additional water-soluble film-forming polymer.

20. Use according to any one of the preceding claims, **characterized in that** the composition comprises an additional wax in the form of particles having a size of greater than or equal to 1 pm dispersed in the aqueous phase.

21. Use according to any one of the preceding claims, **characterized in that** the composition comprises, in addition, an additive chosen from the group formed by pigments, pearlescent agents, fillers, trace elements, demulcents, sequestrants, perfumes, oils, silicones, thickeners, vitamins, proteins, ceramides, plasticizers, coalescing agents, cohesion agents, alkalinizing agents, acidifying agents, emollients and preservatives.

22. Mascara comprising an aqueous dispersion of particles of film-forming polymer, the said particles of film-forming polymer having a size ranging from 10 to 500 nm, **characterized in that** it comprises an aqueous microdispersion of particles of wax, and the said film-forming polymer not being a colouring polymer.

23. Mascara according to Claim 22, **characterized in that** the particles of film-forming polymer have a size ranging from 20 to 300 nm.

24. Mascara according to Claim 22 or 23, **characterized in that** the film-forming polymer in aqueous dispersion is chosen from free-radical polymers, polycondensates and polymers of natural origin.

25. Mascara according to any one of Claims 22 to 24, **characterized in that** the film-forming polymer is chosen from vinyl polymers, polyurethanes, polyesters and polyamides.

26. Mascara according to any one of Claims 22 to 25, **characterized in that** the film-forming polymer in aqueous dispersion is a polyurethane.

27. Mascara according to any one of Claims 22 to 26, **characterized in that** the film-forming polymer has a water uptake of less than or equal to 50%, preferably of less than or equal to 40% and better still of less than or equal to 30%.

28. Mascara according to any one of Claims 22 to 27, **characterized in that** the film-forming polymer in aqueous dispersion is present in an amount ranging from 1% to 60% by weight, relative to the total weight of the composition, preferably from 5% to 40% by weight, and better still from 10% to 30% by weight.

29. Mascara according to any one of Claims 22 to 28, **characterized in that** the microdispersion of wax comprises particles of waxes having a mean size of less than 1 μm, preferably of less than 0.5 μm.

30. Mascara according to any one of Claims 22 to 29, **characterized in that** the wax has a melting point ranging from 30°C to 120°C, and preferably ranging from 45°C to 120°C.

31. Mascara according to any one of Claims 22 to 30, **characterized in that** the wax of the microdispersion is a polar wax.

32. Mascara according to any one of Claims 22 to 31, **characterized in that** the wax is chosen from the group formed by beeswax, lanolin wax, Chinese waxes; rice wax, Carnauba wax, candelilla wax, ouricury wax, cork fibre wax, sugarcane wax, Japan wax and sumac wax; montan wax, the waxes obtained by catalytic hydrogenation of animal or vegetable oils having linear or branched $C_8$-$C_{32}$ fatty chains; hydrogenated jojoba oil, hydrogenated sunflower oil, hydrogenated castor oil, hydrogenated copra oil, hydrogenated lanolin oil; silicone waxes; mixtures thereof.

33. Mascara according to any one of Claims 22 to 32, **characterized in that** the wax has a hardness ranging from 0.05 MPa to 15 MPa, and preferably ranging from 6 MPa to 15 MPa.

34. Mascara according to any one of Claims 22 to 33, **characterized in that** the wax of the said microdispersion is present in a dry matter content ranging from 0.1% to 50% by weight, relative to the total weight of the composition, preferably ranging from 1% to 30% by weight, and better still ranging from 5% to 20% by weight.

**35.** Mascara according to any one of Claims 22 to 34, in which the particles of the microdispersion of wax comprise, in addition, oily and/or pasty fatty additives and/or a fat-soluble additive/active agent.

**36.** Mascara according to any one of Claims 22 to 35, comprising, in addition, at least one surfactant.

**37.** Mascara according to any one of Claims 22 to 36, **characterized in that** the film-forming polymer in aqueous dispersion and the microdispersion of wax are present in the composition in a film-forming polymer/microdispersed wax weight ratio ranging from 50/50 to 95/5, preferably 60/40 to 80/20.

**38.** Mascara according to any one of Claims 22 to 37, **characterized in that** the mascara comprises, in addition, an additional water-soluble film-forming polymer.

**39.** Mascara according to any one of Claims 22 to 38, **characterized in that** it comprises, in addition, an additive chosen from the group formed by pigments, pearlescent agents, fillers, trace elements, demulcents, sequestrants, perfumes, oils, silicones, thickeners, vitamins, proteins, ceramides, plasticizers, coalescing agents, cohesion agents, alkalinizing agents, acidifying agents, emollients and preservatives.

**40.** Method for the application of make-up to or the nontherapeutic care of keratinous fibres, in particular the eyelashes, **characterized in that** it comprises the application, to the keratinous fibres, of a mascara according to any one of Claims 22 to 39.